(19) Europäisches Patentamt
European Patent Office
Office européen des brevets

(11) Numéro de publication: 0 322 296 B1

(12) # FASCICULE DE BREVET EUROPEEN

(45) Date de publication de fascicule du brevet: **17.03.93**

(51) Int. Cl.[5]: **C07D 277/28**, C07D 277/32, A61K 31/425

(21) Numéro de dépôt: **88403233.5**

(22) Date de dépôt: **19.12.88**

(54) **Dérivés oxyimino du thiazole, procédé de préparation et utilisation en thérapeutique.**

(30) Priorité: **23.12.87 FR 8718056**

(43) Date de publication de la demande:
**28.06.89 Bulletin 89/26**

(45) Mention de la délivrance du brevet:
**17.03.93 Bulletin 93/11**

(84) Etats contractants désignés:
**AT BE CH DE ES FR GB GR IT LI LU NL SE**

(56) Documents cités:
**GB-A- 2 056 440**

**CHEMICAL ABSTRACTS, vol. 48, no. 6, 25 mars 1954, colonne 3295, Columbus, Ohio, US; S. MINORU et al.: "Chloramphenicol analogs. IV. Synthesis of DL-1-(4-methyl-5-thiazolyl)-2-acetamido-1,3-propanediol"**

(73) Titulaire: **INSTITUT DE RECHERCHES CHIMIQUES ET BIOLOGIQUES APPLIQUEES (I.R.C.E.B.A.) Société à responsabilité limitée dite:**
**62, Grande-Rue**
**F-78490 Vicq(FR)**

(72) Inventeur: **Danree, Bernard**
**59 bis, Boulevard Deveaux**
**F-78300 Poissy(FR)**
Inventeur: **Houziaux, Patrick**
**Chemin des Jonchères/Bazemont**
**F-78580 Maule(FR)**
Inventeur: **Lacolle, Jean-Yves**
**27, Route des Suisses**
**F-78170 La Celle Saint-Cloud(FR)**
Inventeur: **Riffaud, Jean-Pierre**
**108, Avenue des Etats-Unis**
**F-78000 Versailles(FR)**

(74) Mandataire: **Clisci, Serge et al**
**S.A. FEDIT-LORIOT CONSEILS EN PROPRIETE INDUSTRIELLE 38, avenue Hoche**
**F-75008 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 322 296 B1

## Description

### DOMAINE DE L'INVENTION

La présente invention concerne, en tant que produits industriels nouveaux, des dérivés oxyimino du thiazole, à savoir les 5-(1-alkoxyimino- et 1-hydroxyimino-éthyl)-4-méthylthiazoles de formule I ci-après et leurs sels d'addition. Elle concerne également le procédé de préparation et l'utilisation en thérapeutique de ces produits, notamment en tant qu'agents anticonvulsivants.

### ART ANTERIEUR

On sait que de nombreux dérivés de thiazole ont été décrits dans le passé et qu'un nombre restreint de ces dérivés présente des propriétés anticonvulsivantes.

On sait, en particulier des documents FR-A-945 198, GB-A-641 426 et US-A-2 654 760, que le 5-(2-hydroxyéthyl)-4-méthylthiazole est un intermédiaire de synthèse utile dans la préparation de la vitamine B1 ; et on sait, notamment du résumé des Chemical Abstracts 74, 141 617 w, que son isomère le 1-(4-méthyl-5-thiazolyl)-1-éthanol possède des propriétés anticonvulsivantes.

On sait, notamment du document FR-A-2 555 583, que le 2-(4-méthyl-5-thiazolyl)-2-propanol présente des propriétés anticonvulsivantes et antihypoxiques alors que l'un de ses homologues inférieurs, le 5-hydroxyméthyl-4-méthylthiazole, est dépourvu d'effets anticonvulsivants et que son autre homologue inférieur, le 1-(4-méthyl-5-thiazolyl)-1-éthanol sus-visé, qui est anticonvulsivant, est dépourvu d'effets antihypoxiques bénéfiques.

On connaît également de l'article de M. SUZUKI et al., J. Pharm. Soc. Japan 73, 394-396 (1953), résumé dans Chemical Abstracts, 48, 3295c-h (1954), le 5-(1-hydroximinoéthyl)-4-méthylthiazole. Toutefois cet article ne décrit ni ne suggère les éventuelles propriétés pharmacologiques de ce produit et par suite n'envisage pas son utilisation en thérapeutique en tant que moyen anticonvulsivant.

On connaît par ailleurs de la demande de brevet français de la Demanderesse déposée le 29 juillet 1987 sous le No. 87 10 738 des dérivés de 2-pipérazinoacétamido-4-méthylthiazole utiles en tant qu'agents anticonvulsivants.

BUT ET OBJET DE L'INVENTION

Le but principal de l'invention est de fournir de nouveaux dérivés du thiazole qui sont (i) structurellement différents des composés de l'art antérieur sus-visé, et (ii) utiles en thérapeutique eu égard à leurs propriétés anticonvulsivantes.

Selon l'invention, on propose également un procédé de préparation de ces nouveaux dérivés ainsi que leur utilisation en thérapeutique vis-à-vis des convulsions, notamment les convulsions d'origine épileptique.

Les nouveaux dérivés oxyimino du thiazole selon l'invention sont caractérisés en ce qu'ils sont choisis parmi l'ensemble comprenant

(i) les 5-(1-alkoxyimino- et 1-hydroxyimino-éthyl)-4-méthylthiazoles de formule

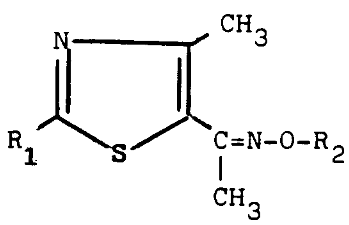

(I)

dans laquelle $R_1$ représente H, F, Cl, Br, ou $CH_3$; et, $R_2$ représente un groupe alkyle en $C_1$-$C_{10}$, un groupe benzyle de formule $CH_2C_6H_2(R_3)_3$,

où chaque groupe $R_3$, identique ou différent, représente H, F, Cl, Br, $CF_3$, un groupe alkyle en $C_1$-$C_4$ ou un groupe alkoxy en $C_1$-$C_4$,

un groupe $CH_2COOR_4$,

où $R_4$ représente H, un groupe alkyle en $C_1$-$C_4$ ou le groupe ammonium $NH_4^+$,

un groupe $CH_2COO1/m$ M

où M est un métal alcalin ou alcalino-terreux et m sa valence,

ou un groupe 2-aminoéthyle de formule $CH_2CH_2NR_5R_6$

où $R_5$ et $R_6$, qui peuvent être identiques ou différents, représentent chacun H ou un groupe alkyle en $C_1$-$C_4$, $R_5$ et $R_6$ considérés ensemble avec l'atome d'azote auquel ils sont lies pouvant former un groupe N-hétérocyclique saturé de 5 à 7 sommets et "choisi parmi l'ensemble constitué par les groupes pyrrolidino, pipéridino, morpholino, thiomorpholino, et hexaméthylèneimino,

$R_2$ pouvant également représenter l'atome d'hydrogène quand $R_1$ est différent de H ; et,

(ii) leurs isomères géométriques de constitution ; et,

(iii) les sels d'addition correspondants.

Dans son aspect le plus large, l'invention concerne l'utilisation en tant que médicaments anticonvulsivants des produits sus-visés, d'une part, et du 5-(1-hydroxyiminoéthyl)-4-méthylthiazole composé de formule I où $R_1$ = $R_2$ = H, qui a été décrit en tant que curiosité de laboratoire sans mention de ses éventuelles propriétés pharmaceutiques dans l'article de M. SUZUKI précité, et de celles de ses isomères E, Z et des sels d'addition correspondants, d'autre part.

## DESCRIPTION DETAILLEE DE L'INVENTION

L'invention concerne donc les isomères géométriques E et Z de formule I et leurs mélanges, d'une part, et les sels d'addition desdits isomères géométriques et de leurs mélanges, d'autre part.

L'existence d'isomères E et/ou Z traduit les relations stériques par rapport à la liaison non-cyclique C=N ;
pour les configurations E (de l'allemand "entgegen", i.e. "opposé") et Z (de l'allemand "zusammen", i.e. "ensemble"), on compare le groupe prioritaire lié à l'un des atomes de la double liaison au groupe prioritaire lié à l'autre atome de ladite double liaison, à savoir dans le cas d'espèce le groupe 5-thiazolyle sur l'atome de carbone, et respectivement, le groupe $OR_2$ sur l'atome d'azote.

Par sels d'addition, on entend ici les sels d'addition d'acide obtenus par réaction d'une base libre de formule I, avec un acide minéral ou organique. Parmi les acides utilisables pour salifier les bases libres de formule I, on peut notamment mentionner les acides chlorhydrique, bromhydrique, acétique, formique, propionique, succinique, cinnamique, citrique, méthanesulfonique, p-toluènesulfonique. D'une manière générale, les chlorhydrates sont ici les sels d'addition préférés pour l'utilisation thérapeutique en tant que moyens anticonvulsivants.

Les groupes alkyle qui interviennent dans la définition de $R_2$ sont des restes hydrocarbonés en $C_1$-$C_{10}$, de préférence un groupe alkyle inférieur en $C_1$-$C_4$, à chaîne linéaire ou ramifiée. A titre d'exemple lorsque $R_2$ représente un groupe alkyle il peut être notamment un groupe méthyle, éthyle, n-propyle, i-propyle, n-butyle, s-butyle, t-butyle ou 2-propylpentyle. Les groupes alkyle préférés pour $R_2$ sont $CH_3$, $C_2H_5$, i-$C_3H_7$ et n-$C_4H_9$.

De même, les groupes alkyle qui interviennent dans les définitions de $R_3$, $R_4$, $R_5$ et $R_6$ sont des restes hydrocarbonés en $C_1$-$C_4$ à chaîne linéaire ou ramifiée. Les groupes alkyle préférés pour $R_3$ et $R_4$ sont les groupes éthyle et surtout méthyle, et, pour $R_5$ et $R_6$ les groupes t.-butyle, i-propyle, éthyle et surtout méthyle.

Les groupes alkoxy qui interviennent dans la définition de $R_3$ comportent des restes hydrocarbonés en $C_1$-$C_4$ à chaîne linéaire ou ramifiée qui sont monovalents et liés à un atome d'oxygène. Conviennent notamment les groupes méthoxy, éthoxy, n-propyloxy, i-propyloxy, s.-butyloxy, i-butyloxy, t.-butyloxy et n-butyloxy. Les groupes alkoxy préférés pour $R_3$ sont les groupes méthoxy et surtout éthoxy.

Les groupes N-hétérocycliques qui interviennent dans la définition de $NR_5R_6$ sont choisis parmi l'ensemble constitué par les groupes pyrrolidino, pipéridino, morpholino, thiomorpholino, et hexaméthylèneimino. Il s'agit de groupes N-hétérocycliques saturés c'est-à-dire dépourvus de double liaison C=C ou C=N et comportant de 5 à 7 sommets. Les groupes N-hétérocycliques les plus intéressants sur le plan pharmacologique sont les groupes pipéridino, morpholino et mieux pyrrolidino.

Parmi les métaux M qui conviennent, on peut notamment mentionner Na, K et Ca, le sodium étant ici le métal préféré.

Les composés nouveaux, qui sont préférés selon l'invention, sont, eu égard à ce qui précède, les composés de formule I dans laquelle $R_1$ est H, Cl, Br ou $CH_3$, et $R_2$ est $CH_3$, $C_2H_5$, i-$C_3H_7$, n-$C_4H_9$, $CH_2C_6H_2(R_3)_3$ (où chaque groupe $R_3$, identique ou différent, est H, F, Cl, Br, $CF_3$, $CH_3$, $CH_3O$ ou $C_2H_5O$), $CH_2COONa$, $CH_2COOK$, $CH_2COOCH_3$, $CH_2COOC_2H_5$, $CH_2CH_2N(CH_3)_2$, $CH_2CH_2N(C_2H_5)_2$, 2-pyrrolidinoéthyle, 2-morpholinoéthyle ou 2-pipéridinoéthyle, $R_2$ pouvant représenter H lorsque $R_1$ est différent de H.

Parmi ceux-ci, les plus intéressants du point de vue pharmacologique sont (i) les composés de formule I où $R_1$ est H et $R_2$ est $CH_3$, $C_2H_5$, i-$C_3H_7$, n-$C_4H_9$, $CH_2C_6H_4R_3$ (où $R_3$ est H, F, Cl, Br, $CF_3$, $CH_3$, $CH_3O$ ou $C_2H_5O$), $CH_2COONa$, $CH_2COOCH_3$, $CH_2COOC_2H_5$, $CH_2CH_2N(CH_3)_2$, $CH_2CH_2N(C_2H_5)_2$ ou 2-pyrrolidinoé-

thyle, et
(ii) les composés de formule I où $R_1$ est Cl, Br ou $CH_3$ et $R_2$ est H ou $CH_3$.

Est également intéressant du point de vue phamacologique :
(iii) le composé de formule I où $R_1 = R_2 = H$, décrit comme curiosité de laboratoire par M. SUZUKI et al., dans l'article précité.

Les composés de formule I peuvent être préparés selon une méthode connue en soi par application de mécanismes réactionnels classiques. Le procédé que l'on préconise suivant l'invention, consiste à faire réagir un composé 5-acétyl-4-méthyl-thiazole de formule

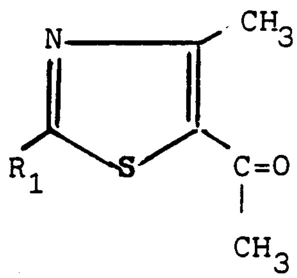

(II)

avec un dérivé d'hydroxylamine de formule :

$H_2NOR_2$ (III)

où $R_1$ et $R_2$ sont définis code indiqué ci-dessus.

Cette réaction est avantageusement réalisée avec un excès de III par rapport aux conditions stoechiométriques. On utilisera avantageusement 1,2 à 1,8 moles (et mieux 1,5 moles) de III pour 1 mole de II, en présence d'acétate de sodium, à une température de l'ordre de 10 à 25 °C [notamment à la température ambiante (15-20 °C)] pendant au moins 3 h, pour mettre en oeuvre la réaction II + III.

L'acétate de sodium, qui intervient dans cette réaction, sera avantageusement utilisé en excès par rapport au composé II. En pratique, on utilisera 1,2 à 1,8 moles et mieux 1,5 moles de $CH_3CO_2Na$ anhydre pour 1 mole de II. En d'autres termes on fera appel à une quantité molaire de $CH_3CO_2Na$ sensiblement identique à celle du dérivé d'hydroxylamine de formule III.

Si nécessaire, le produit de formule I où $R_2$ = H, obtenu selon ladite réaction II + III lorsque le composé III est l'hydroxylamine, peut être soumis à une réaction d'O-alkylation au moyen d'un alcool en $C_1$-$C_4$ [réaction dite de MITSUNOBU décrite par S. BITTNER et al., dans J. Chem. Soc. Perkin I, 1708, (1976)] afin de conduire à un composé de formule I où $R_2$ est différent de l'atome d'hydrogène.

Le mécanisme réactionnel est alors le suivant :

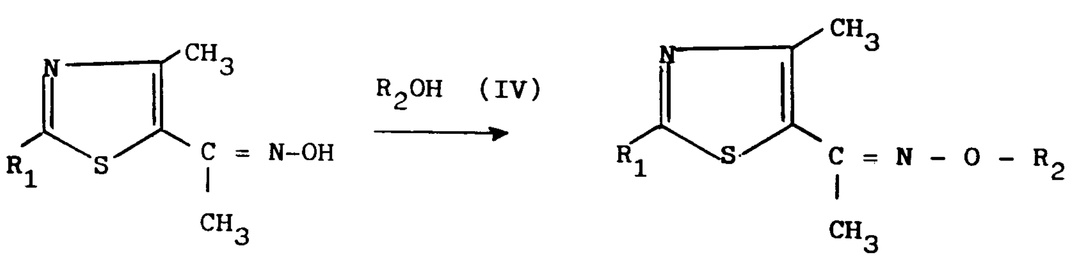


(I') (Ia)

où $R_1$ et $R_2$ sont définis comme indiqué ci-dessus avec la différence que $R_2$ est différent de H.

La réaction I' + IV est avantageusement mise en oeuvre en faisant appel à une quantité d'alcool IV en excès par rapport aux conditions stoechiométriques : l'alcool $R_2OH$ intervenant comme réactif, d'une part, et comme solvant ou co-solvant, d'autre part.

En pratique, cette réaction est réalisée en présence d'une phosphine (notamment la triphénylphosphine) et d'un azodicarboxylate (notamment l'azodicarboxylate de diéthyle), à une température comprise entre 10

et 25 ° C [notamment à la température ambiante (15-20 ° C)] pendant au moins 5 h.

De façon encore plus avantageuse, on utilisera environ 1,5 moles de triphénylphosphine et environ 2 moles d'azodicarboxylate de diéthyle pour 1 mode de composé hydroxyimino I', et l'alcool $R_2OH$ sera un alcool primaire ou secondaire.

En résumé, la réaction II + III = I est celle que l'on préconise suivant l'invention en ce sens qu'elle s'applique à la synthèse de tous les composés de formule I. Les composés alkoxyimino de formule I (où $R_2$ est différent de H) peuvent être préparés selon la réaction générale II + III = I sus-visée, d'une part, ou selon la réaction particulière I' + IV = $I_a$, d'autre part.

Compte tenu de la méthode de synthèse utilisée, le composé de formule I selon l'invention est en général un mélange des isomères E et Z. Les isomères E et Z peuvent être isolés dudit mélange selon une méthode connue en soi, notamment par distillation ou cristallisation fractionnées, soit plus avantageusement par chromatographie sur colonne.

On a consigné, dans le tableau I ci-après, un certain nombre de composés typiques selon l'invention. Les points de fusion qui ont été mentionnés dans ce tableau sont, des points de fusion instantanée déterminés au banc Kofler.

La configuration des produits des exemples 1-9 et 11-29 a été déterminée [voir à cet effet le rapport pondéral (et molaire) du mélange E/Z dans ledit tableau I]. La configuration du produit de d'exemple 10 n'ayant pas été étudiée, on présume qu'il s'agit plutôt d'un mélange d'isomères E et Z que l'un desdits isomères E ou Z pur; très vraisemblablement pour ledit produit de l'exemple 10 on présume que le rapport E/Z est supérieur ou égal à 80/20 comme pour les autres produits du tableau I.

L'analyse du produit de d'exemple 2 montre qu'il s'agit d'un mélange E/Z dans un rapport pondéral (et molaire) de 90/10. Le produit de l'exemple 3 est l'isomère E pur qui a été obtenu à partir du produit de l'exemple 2 par chromatographie sur colonne.

**TABLEAU I**

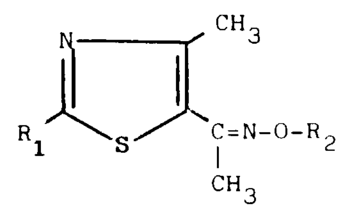

(I)

| Produit | Code No. | $R_1$ | $R_2$ | E/Z (%) | F(°C) ou $n_D$ |
|---|---|---|---|---|---|
| Ex 1 (a) | B-1222 | H | $CH_3$ | 91/9 | $n_D^{25°C}$ = 1,5528 (e) |
| Ex 2 (b) | B-1044 | H | $CH_3$ | 90/10 | F = 100-105 (f) |
| Ex 3 (a,d) | B-1234 | H | $CH_3$ | 100/0 | F = 90-95 (f) |
| Ex 4 (a) | B-1229 | H | $CH_2CH3$ | 81/19 | $n_D^{21°C}$ = 1,5395 (g) |
| Ex 5 (b) | B-1235 | H | $CH2CH_3$ | 85/15 | F = 95-100 (f) |
| Ex 6 (a) | B-1264 | H | $CH(CH_3)_2$ | 86/14 | $n_D^{21°C}$ = 1,5285 |

**TABLEAU I (continuation 1)**

| Produit | Code No. | $R_1$ | $R_2$ | E/Z (%) | F(°C) ou $n_D$ |
|---|---|---|---|---|---|
| Ex 7 (b) | B-1265 | H | $CH(CH_3)_2$ | 86/14 | F = 100-103 (f) |
| Ex 8 (a) | B-1266 | H | $(CH_2)_3CH_3$ | 89/11 | $n_D^{21°C}$ = 1,5275 |
| Ex 9 (b) | B-1233 | H | $(CH_2)_3CH_3$ | 98/2 | F = 68-73 (h) |
| Ex 10 (a) | B-1384 | H | $CH_2CH(C_3H_7)_2$ | - | - |
| Ex 11 (a) | B-1255 | H | 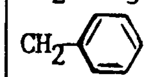 | 89/11 | $n_D^{21°C}$ = 1,5919 (i) |
| Ex 12 (b) | B-1262 | H | 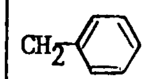 | 98/2 | F = 103-104 (j) |
| Ex 13 (a) | B-1375 | H | 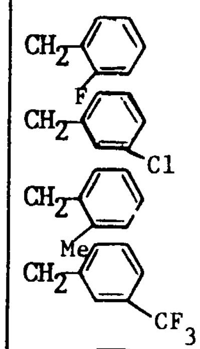 | 85/15 | $n_D^{20°C}$ = 1,5772 (k) |
| Ex 14 (a) | B-1376 | H | | 82/18 | $n_D^{21°C}$ = 1,5960 (l) |
| Ex 15 (a) | B-1379 | H | | 87/13 | $n_D^{21°C}$ = 1,5909 (y) |
| Ex 16 (a) | B-1377 | H | | 85/15 | $n_D^{21,5°C}$ = 1,5370 (z) |
| Ex 17 (a) | B-1378 | H | 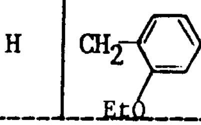 | 85/15 | $n_D^{20°C}$ = 1,5809 (aa) |

**TABLEAU I (continuation 2)**

| Produit | Code No. | $R_1$ | $R_2$ | E/Z (%) | F(°C) ou $n_D$ |
|---|---|---|---|---|---|
| Ex 18 (a) | B-1290 | H | $CH_2COOCH_3$ | 91/9 | $n_D^{21°C}$ = 1,5385 (m) |
| Ex 19 (a,n) | B-1329 | H | $CH_2COONa$ | 99,5/0,5 | F = 205-207 (o) |
| Ex 20 (c,n) | B-1296 | H | $CH_2CH_2N(CH_3)_2$ | 98/2 | F = 160-165 (p) |
| Ex 21 (a) | B-1304 | H | $CH_2CH_2$- 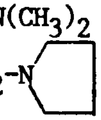 | 98/2 | $n_D^{22°C}$ = 1,5502 (q) |
| Ex 22 (a) | B-1097 | Cl | H | 97/3 | F = 141-142 (r) |
| Ex 23 (a) | B-1077 | Br | H | 85/15 | F = 149-150 (s) |
| Ex 24 (a) | B-1193 | Cl | $CH_3$ | 90/10 | $n_D^{21°C}$ = 1,5617 (t) |
| Ex 25 (a) | B-1267 | Br | $CH_3$ | 80/20 | (u) |
| Ex 26 (a) | B-1061 | $CH_3$ | $CH_3$ | 98/2 | $n_D^{25,6°C}$ = 1,5408 |
| Ex 27 (b) | B-1042 | $CH_3$ | $CH_3$ | 98/2 | F = 130-135 (o) |
| Ex 28 (a,v) | B-876 | H | H | 99/1 | F = 142-143 (w) |
| Ex 29 (b) | B-1026 | H | H | 99/1 | F = 185-188 (x) |

**TABLEAU I (continuation 3)**

Notes

(a) : base libre
(b) : monochlorhydrate
(c) : dichlorhydrate
(d) : isomère E pur
(e) : $Eb_{0,22\ mmHg}$= 72-74°C

(f) : solvant de recristallisation : AcOEt
(g) : $Eb_{0,20\ mmHg}$= 58-62°C

(h) : solvant de recristallisation : mélange hexane/AcOEt (1/1)v/v
(i) : $Eb_{0,10\ mmHg}$= 130-132°C

(j) : solvant de recristallisation : AcOEt

(k) : $Eb_{0,15\ mmHg}$= 110-115°C

(l) : $Eb_{0,10\ mmHg}$= 125-130°C

(m) : $Eb_{0,15\ mmHg}$= 98°C environ

(n) : monohydrate
(o) : solvant de recristallisation AcOEt/isopropanol (1/1) v/v
(p) : solvant de recristallisation AcOEt/EtOH (1/1) v/v
(q) : $Eb_{0,20\ mmHg}$= 108-110°C

.../...

**TABLEAU I (fin)**

Notes

(r) : solvant de recristallisation : n-butanol

(s) : solvant de recristallisation : toluène

(t) : $Eb_{0,15\ mmHg}$ = 70-72°C

(u) : $Eb_{0,12\ mmHg}$ = 82-83°C

(v) : décrit en tant que produit par M. SUZUKI et al.

(w) : solvant de recristallisation : MeOH

(x) : solvant de recristallisation : isopropanol

(y) : $Eb_{0,1\ mmHg}$ = 128-134°C

(z) : $Eb_{0,15\ mmHg}$ = 110-115°C

(aa) : $Eb_{0,1\ mmHg}$ = 134-138°C

Remarques :

Les valeurs de pression 0,10 mmHg, 0,12 mmHg, 0,15 mmHg, 0,20 mmHg et 0,22 mmHg correspondent respectivement à environ 13,3 Pa, 15,9 Pa, 19,9 Pa, 26,6 Pa et 29,3 Pa.

Le meilleur mode de mise en oeuvre de l'invention consiste à utiliser de 5-(1-méthoxyiminoéthyl)-4-méthylthiazole, le 5-(1-benzyloxyiminoéthyl)-4-méthylthiazole et leurs sels d'addition non-toxiques en tant que produits industriels nouveaux utiles en thérapeutique; les produits les plus intéressants étant a) de mélange E/Z = 91/9 du dérivé méthoxyimino sus-visé (base libre de l'exemple 1 ; No de Code : B-1 222), b) de mélange E/Z = 90/10 du chlorhydrate correspondant (monochlorhydrate de l'exemple 2 ; No de Code : B-1 044) ; c) le mélange E/Z : 89/11 du dérivé benzyloxyimino sus-visé (base libre de l'exemple 11 ; No

de Code : B-1 255), et d) de mélange E/Z = 98/2 du chlorhydrate correspondant (monochlorhydrate de l'exemple 12 ; No de Code : B-1 262). L'isomère E pur (rapport E/Z = 100/0) dudit dérivé méthoxyimino (base libre de l'exemple 3 ; No de Code B-1 234) est également intéressant mais est moins actif en tant qu'agent anticonvulsivant que de mélange E/Z = 91/9 correspondant.

Les composés selon d'invention, à savoir les isomères E et Z et leurs mélanges de formule I bis, ci-après, d'une part, et leurs sels d'addition, d'autre part, sont utiles en thérapeutique. Ils agissent en tant qu'ingrédients actifs anticonvulsivants et sont donc recommandés dans le traitement des convulsions et notamment des convulsions d'origine épileptique chez l'homme.

Suivant l'invention, on préconise donc une composition thérapeutique, caractérisée en ce qu'elle renferme, en association avec un excipient physiologiquement acceptable, au moins un composé choisi parmi (a) les isomères géométriques E et Z et leurs mélanges de formule

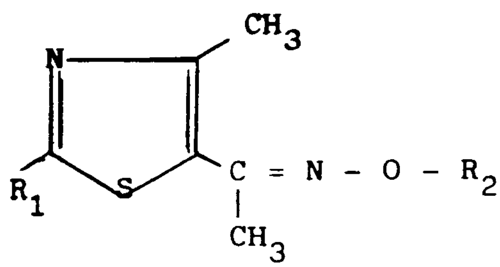

(I bis)

où $R_1$ représente H, F, Cl, Br ou $CH_3$, et $R_2$ représente H, un groupe alkyle en $C_1$-$C_{10}$, notamment un groupe alkyle inférieur en $C_1$-$C_4$,
un groupe benzyle de formule $CH_2C_6H_2(R_3)_3$,
où chaque $R_3$, identique ou différent, représente H, F, Cl, Br, $CF_3$, un groupe alkyle en $C_1$-$C_4$ ou un groupe alkoxy en $C_1$-$C_4$,
un groupe $CH_2COOR_4$,
où $R_4$ représente H, un groupe alkyle en $C_1$-$C_4$ ou le groupe ammonium $NH_4^+$,
un groupe $CH_2COO^1/m$ M,
où M est un métal alcalin ou alcalino-terreux et m sa valence,
ou un groupe 2-aminoéthyle de formule $CH_2CH_2NR_5R_6$,
où $R_5$ et $R_6$, qui peuvent être identiques ou différents, représentent chacun H ou un groupe alkyle en $C_1$-$C_4$, $R_5$ et $R_6$ considérés ensemble avec l'atome d'azote auquel ils sont liés pouvant former un groupe N-hétérocyclique saturé de 5 à 7 sommets et choisi parmi l'ensemble constitué par des groupes pyrrolidino, pipéridino, morpholino, thiomorpholino, et hexaméthylèneimino; et,
(b) leurs sels d'addition non-toxiques.

Bien entendu, dans une telle composition le principe actif intervient en quantité thérapeutiquement efficace.

Suivant l'invention, on préconise également une utilisation thérapeutique suivant laquelle on prépare, à partir d'une substance choisie parmi les isomères E et Z des 5-(1-alkoxyimino- et 1-hydroxyimino-éthyl)-4-méthylthiazoles de formule I bis et leurs mélanges, d'une part, et leurs sels d'addition non-toxiques, d'autre part, un médicament anticonvulsivant destiné à une utilisation thérapeutique vis-à-vis des convulsions notamment les convulsions d'origine épileptique.

D'autres avantages et caractéristiques de l'invention seront mieux compris à la lecture de la description, qui va suivre, d'exemples de préparation et de résultats d'essais toxicologiques et pharmacologiques. L'ensemble de ces éléments est donné à titre d'illustration.

PREPARATION I

Obtention du 5-(1-méthoxyiminoéthyl)-4-méthylthiazole

(Exemple 1 ; No. de Code : B-1222).

Dans un tricol de quatre litres, muni d'un réfrigérant, d'un thermomètre, d'une ampoule d'introduction et d'une agitation pneumatique, on introduit 141,18 g (1 mole) de 5-acétyl-4-méthylthiazole et 1 410 ml de méthanol. A la solution ainsi obtenue, on ajoute goutte à goutte 125,28 g (1,5 moles) de chlorhydrate de méthyloxyamine dissous dans 250 ml d'eau. L'agitation est poursuivie durant 0,5 h puis on ajoute par

portions au mélange résultant 123,06 g (1,5 modes) d'acétate de sodium anhydre.

Le milieu réactionnel ainsi obtenu est laissé sous agitation à température ambiante (15-20 ° C) pendant 12 h, puis filtré sur verre fritté pour éliminer le chlorure de sodium formé. Le filtrat recueilli est concentré à sec sous vide ; le résidu d'évaporation ainsi obtenu est repris par du chlorure de méthylène. L'insoluble est écarté par filtration sur verre fritté (élimination des sels restants). Le filtrat est concentré à sec sous vide pour donner 170 g d'un produit brut que l'on purifie par distillation fractionnée sous vide. On obtient 157,4 g (rendement : 92,5 %) du produit annoncé qui se présente sous la forme d'une huile jaune.

$$n_D^{25^\circ C} = 1,5528$$

$Eb_{0,22mmHg} = 72\text{-}74\,^\circ C$

(Remarque : 0,22 mm Hg correspond approximativement à 29,3 Pa)

Les analyses (i) par chromatographie sur couche mince (C.C.M.) au moyen de gel de silice et d'une phase mobile constituée d'un mélange cyclohexane-acétate d'éthyle 80/20 v/v, et (ii) par chromatographie en phase vapeur (C.P.V.), ont permis de mettre en évidence que de produit de d'exemple 1 (No. de Code : B-1222) est constitué par un mélange des isomères géométriques E/Z dans le rapport pondéral et molaire de 91/9.

PREPARATION II

Obtention du chlorhydrate de 5-(1-méthoxyiminoéthyl)-4-méthylthiazole

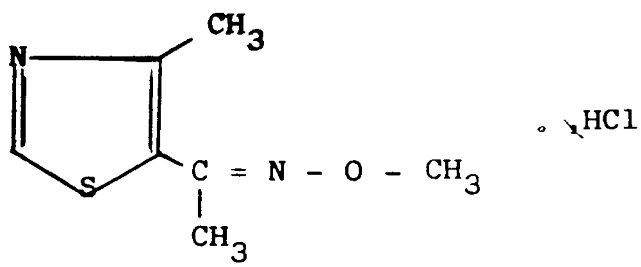

(Exemple 2 ; No. de Code : B-1044)

On dissout 17,02 g (0,1 mode) de B-1222 obtenu selon le procédé de la préparation I ci-dessus, dans 200 ml de diéthyléther anhydre. La solution résultante refroidie est saturée par un courant gazeux d'HCl sec. Les cristaux formés sont filtrés sur verre fritté, lavés par du diéthyléther anhydre, puis séchés sous vide à 60 ° C. Par recristallisation dans l'acétate d'éthyle on obtient 18 g (rendement : 87 %) du produit attendu se présentant sous la forme de cristaux blancs.

| | |
|---|---|
| $F_{inst}$ = | 100 - 105 ° C |
| Spectre I.R. : | conforme à la structure proposée |
| Spectre R.M.N. : | conforme à la structure proposée |
| C.C.M. : | 2 spots (isomères E et Z) |
| C.P.V. : | 2 pics (isomères E et Z) |

Les analyses (i) par chromatographie sur couche mince (C.C.M.) au moyen de gel de silice et d'une phase mobile constituée d'un mélange cyclohexane-acétate d'éthyle 80/20 v/v, et (ii) par chromatographie en phase vapeur (C.P.V.), ont permis de mettre en évidence que de produit de l'exemple 2 (No. de Code : B-1 044) est constitué par un mélange des isomères géométriques E/Z dans de rapport molaire 90/10.

**PREPARATION III**

Obtention du 5-(1-n-butoxyiminoéthyl)-4-méthyl-thiazole.

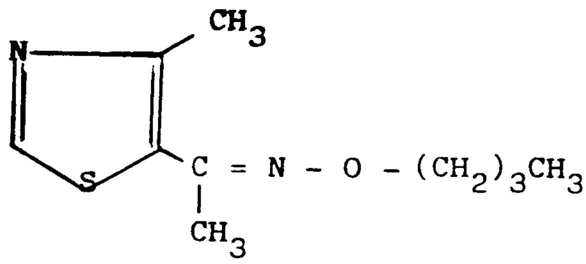

(Exemple 8 ; No de Code : B-1266)

Dans un tricol de 500 ml, muni d'un réfrigérant, d'une ampoule d'introduction, d'un thermomètre et d'une agitation magnétique, on introduit 15,61 g (0,1 mole) de 5-(1-hydroxyiminoéthyl)-4-méthylthiazole et 115 ml de 1-butanol. Au mélange résultant on ajoute 39,38 g (0,15 mole) de triphénylphosphine en solution dans 45 ml de tétrahydrofuranne et 34,86 g (0,2 mole) d'azodicarboxylate de diéthyle en solution dans 65 ml de 1-butanol. Le milieu réactionnel résultant est maintenu sous agitation à température ambiante (15-20 ° C) pendant 36 h. Après avoir écarté l'insoluble par filtration sur verre fritté, on évapore le filtrat sous vide à sec. Le résidu d'évaporation est repris plusieurs fois à l'hexane. Les phases organiques sont rassemblées et concentrées sous vide à sec. On obtient 20,2 g (rendement : 95,1 %) du produit attendu qui se présente sous la forme d'une huile jaune. Le spectre I.R. est conforme à la structure proposée. Il s'agit d'un mélange d'isomères E et Z dans le rapport pondéral et molaire E/Z = 89/11.

$$n_D^{21°C} = 1,5275$$

PREPARATION IV

Obtention du chlorhydrate de 5-(1-n-butoxyiminoéthyl)-4-méthylthiazole.

(Exemple 9 ; No. de Code : B-1233).

La base libre obtenue suivant la préparation III est transformée en sel d'addition, dans le cas d'espèce, en chlorhydrate, selon de procédé décrit dans la préparation II. On obtient, par recristallisation dans le mélange hexane/acétate d'éthyle (1/1)v/v, le B-1233 attendu avec un rendement de 85 % environ.

$F_{inst}$ = 68-73 ° C

Il s'agit d'un mélange d'isomères E et Z dans de rapport pondéral et molaire E/Z = 98/2.

ESSAIS PHARMACOLOGIQUES

On a résumé ci-après un certain nombre d'essais qui ont été entrepris avec les composés suivant l'invention par comparaison avec deux produits anticonvulsivants de référence, à savoir de valproate de sodium (en abrégé VALP) et la triméthadione (en abrégé TRIM).

I - TOXICITE

La toxicité des produits à étudier a été recherchée par administration orale, à l'aide d'une sonde oesophagienne, de chacun desdits produits chez la souris mâle d'un poids corporel de 18 à 22 g et répartie en lots de 10 à 20 animaux chacun. La mortalité a été enregistrée pendant une période de 14 jours.

Les résultats qui ont été obtenus et qui sont exprimés sous forme de DL-50 en mg/kg sont consignés dans le tableau II ci-après.

II - PROPRIETES ANTICONVULSIVANTES

Les propriétés anticonvulsivantes ont été recherchées chez la souris mâle trente minutes après administration orale des substances à étudier, par induction de crises convulsives.

Ces crises sont provoquées, soit par injection intrapéritonéale de pentétrazol (125 mg/kg) soit par stimulation électrique de la cornée (électrochoc supra-maximal; en abrégé : E.C.M.).

Les résultats exprimés sous forme de dose efficace 50 (DE-50) per os, (dose protégeant 50 % des animaux), sont consignés dans le tableau III ci-après. Ils montrent que les produits suivant l'invention exercent un effet protecteur vis-à-vis des convulsions au moins aussi important que celui des deux produits de référence (VALP et TRIM) quand on considère les valeurs des DE-50.

TABLEAU II

| **TOXICITE PER OS CHEZ LA SOURIS*** | | |
|---|---|---|
| Produit | No. de Code | DL-50 mg/kg |
| Ex 1 | B-1222 | 740 |
| Ex 2 | B-1044 | 1000 |
| Ex 3 | B-1234 | 570 |
| Ex 4 | B-1229 | 1000 |
| Ex 5 | B-1235 | 1000 |
| Ex 6 | B-1264 | >1000 |
| Ex 7 | B-1265 | >1000 |
| Ex 8 | B-1266 | 750 |
| Ex 9 | B-1233 | >1000 |
| Ex 11 | B-1255 | >1000 |
| Ex 12 | B-1262 | >1000 |
| Ex 13 | B-1375 | >1000 |
| Ex 14 | B-1376 | >1000 |
| Ex 15 | B-1379 | >1000 |
| Ex 16 | B-1377 | >1000 |
| Ex 17 | B-1378 | >1000 |
| Ex 18 | B-1290 | >1000 |
| Ex 19 | B-1329 | >1000 |
| Ex 20 | B-1296 | >1000 |
| Ex 21 | B-1304 | 360 |
| Ex 22 | B-1097 | >1000 |
| Ex 23 | B-1077 | >1000 |
| Ex 24 | B-1193 | >1000 |
| Ex 25 | B-1267 | >1000 |
| Ex 26 | B-1061 | >1000 |
| Ex 27 | B-1042 | 1000 |
| Ex 28 | B-876 | 1000 |
| Ex 29 | B-1026 | 1000 |
| VALP | - | 977 |
| TRIM | - | 2182 |

Note
* toxicité déterminée sur des lots de 10 à 20 souris mâles chacun.

TABLEAU III

| PROPERTIES ANTICONVULSIVANTES CHEZ LA SOURIS* | | | |
|---|---|---|---|
| Produit | No. de Code | DE-50 vis-à-vis des convulsions induites par | |
| | | PTZ mg/kg | E.C.M. mg/kg |
| Ex 1 | B-1222 | 62 | 84 |
| Ex 2 | B-1044 | 72 | 100 |
| Ex 3 | B-1234 | 91 | 97 |
| Ex 4 | B-1229 | 120 | 150 |
| Ex 5 | B-1235 | 100 | 120 |
| Ex 6 | B-1264 | 112 | >200 |
| Ex 7 | B-1265 | 140 | >200 |
| Ex 8 | B-1266 | >200 | >200 |
| Ex 9 | B-1233 | 138 | 150 |
| Ex 11 | B-1255 | 46 | 78 |
| Ex 12 | B-1262 | 100 | 157 |
| Ex 13 | B-1375 | 105 | 184 |
| Ex 14 | B-1376 | 140 | >200 |
| Ex 15 | B-1379 | 60 | - |
| Ex 16 | B-1377 | 96 | >200 |
| Ex 17 | B-1378 | >200 | >200 |
| Ex 18 | B-1290 | >200 | >200 |
| Ex 19 | B-1329 | >200 | >200 |
| Ex 20 | B-1296 | >200 | >200 |
| Ex 21 | B-1304 | >200 | >200 |
| Ex 22 | B-1097 | >200 | >200 |
| Ex 23 | B-1077 | >200 | >200 |
| Ex 24 | B-1193 | >200 | >200 |
| Ex 25 | B-1267 | >200 | >200 |
| Ex 26 | B-1061 | >200 | >200 |
| Ex 27 | B-1042 | >200 | >200 |
| Ex 28 | B-876 | 126 | >200 |
| Ex 29 | B-1026 | 200 | >200 |
| VALP | - | 244 | 242 |
| TRIM | - | 251 | 500 |

Notes

PTZ : pentétrazol ;

E.C.M. : électrochoc supra-maximal;

* DE-50 en mg/kg déterminées per os sur des lots de dix souris mâles chacun.

III - PROPRIETES NEUROTOXIQUES

Les propriétés neurotoxiques ont été appréciées chez la souris mâle suivant le test dit de "la tige tournante" ("Rota-rod") effectué trente minutes après administration orale des substances à étudier. On observe la chute des animaux (répartis en lots de dix souris mâles chacun par dose et par produit) au cours des deux minutes qui suivent leur installation sur la tige.

Les résultats, qui ont été obtenus, sont exprimés sous la forme de dose neurotoxique 50 (DT-50) per os, dose provoquant la chute de 50 % des animaux. Ces résultats sont consignés dans le tableau IV ci-après, dans lequel, pour comparer les composés selon selon l'invention avec les produits de référence précités (VALP et TRIM), on a également donné les valeurs des rapports DT-50/DE-50 (index de protection) et DL-50/DE-50 (index thérapeutique) dans lesquels les DE-50 sont des doses efficaces 50 vis-à-vis des convulsions induites par le pentétrazol (PTZ) et l'électrochoc supra-maximal (E.C.M.) déterminées comme indiqué ci-dessus (voir notamment de tableau III).

Les résultats du tableau IV montrent clairement que les composés selon l'invention Ex 1 (B-1222), Ex 5 (B-1235), Ex 11 (B-1255), Ex 12 (B-1262), Ex 13 (B-1375) et Ex 16 (B-1377) présentent notamment des index de protection et des index thérapeutiques meilleurs que ceux des produits de référence, le valproate de sodium et la triméthadione. Les produits des plus efficaces suivant l'invention sont de produit de l'exemple 11 (B-1255) et le chlorhydrate correspondant, le produit de l'exemple 12 (B-1262).

**TABLEAU IV**

**ACTIVITE NEUROTOXIQUE**

| Produit | No. de Code | DT-50* mg/kg | Index de protection PTZ | | DL-50 mg/kg | Index thérapeutique PTZ | |
|---|---|---|---|---|---|---|---|
| | | | PTZ | E.C.M. | | PTZ | E.C.M. |
| Ex 1 | B-1222 | 347 | 5,6 | 4,1 | 740 | 11,9 | 8,8 |
| Ex 2 | B-1044 | 270 | 3,8 | 2,7 | 1000 | 13,9 | 10,0 |
| Ex 3 | B-1234 | 290 | 3,2 | 3,0 | 570 | 6,3 | 5,9 |
| Ex 5 | B-1235 | 750 | 7,5 | 6,3 | 1000 | 10,0 | 8,3 |
| Ex 9 | B-1233 | 600 | 4,4 | 4,0 | >1000 | >7,3 | >6,7 |
| Ex 11 | B-1255 | 1000 | 21,7 | 12,8 | >1000 | >21,7 | >12,8 |
| Ex 12 | B-1262 | 1353 | 13,5 | 8,6 | >1000 | >10,0 | >6,4 |
| Ex 13 | B-1375 | 750 | 7,1 | 4,0 | >1000 | >9,5 | >5,4 |
| Ex 14 | B-1376 | 750 | 5,4 | - | >1000 | >7,1 | - |
| Ex 16 | B-1377 | 1500 | 15,6 | - | >1000 | >10,4 | - |
| VALP | - | 670 | 2,7 | 2,8 | 977 | 4,0 | 4,0 |
| TRIM | - | 1000 | 4,0 | 2,0 | 2182 | 8,7 | 4,4 |

Notes

Index de protection : DT-50 per os/DE-50 per os
Index thérapeutique : DL-50 per os/DE-50 per os
* dose déterminée per os sur des lots de dix souris mâles chacun.

**Revendications**

**Revendications pour les Etats contractants suivants : AT, BE, CH, DE, FR, GB, IT, LI, LU, NL, SE**

1. Composé oxyimino du thiazole, caractérisé en ce qu'il est choisi parmi l'ensemble comprenant
   (i) les 5-(1-alkoxyimino- et 1-hydroxyimino-éthyl)-4-méthylthiazoles de formule

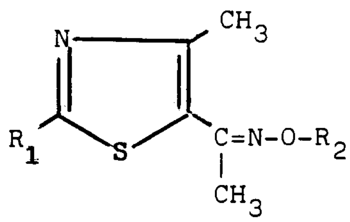

(I)

dans laquelle $R_1$ représente H, F, Cl, Br, ou $CH_3$ ; et,
$R_2$ représente un groupe alkyle en $C_1$-$C_{10}$,
un groupe benzyle de formule $CH_2C_6H_2(R_3)_3$,
où chaque $R_3$, identique ou différent, représente H, F, Cl, Br, $CF_3$ un groupe alkyle en $C_1$-$C_4$ ou un groupe alkoxy en $C_1$-$C_4$,
un groupe $CH_2COOR_4$,
où $R_4$ représente H, un groupe alkyle en $C_1$-$C_4$ ou le groupe ammonium $NH_4^+$,
un groupe $CH_2COO1/m$ M,
où M est un métal alcalin ou alcalino-terreux et m sa valence,
ou un groupe 2-aminoéthyle de formule $CH_2CH_2NR_5R_6$,
où $R_5$ et $R_6$, qui peuvent être identiques ou différents, représentent chacun H ou un groupe alkyle en $C_1$-$C_4$, $R_5$ et $R_6$ considérés ensemble avec l'atome d'azote auquel ils sont liés pouvant former un groupe N-hétérocyclique saturé de 5 à 7 sommets et choisi parmi l'ensemble constitué par les groupes pyrrolidino, pipéridino, morpholino, thiomorpholino, et hexaméthylèneimino
$R_2$ pouvant également représenter l'atome d'hydrogène quand $R_1$ est différent de H ;
(ii) leurs isomères géométriques E et Z ; et,
(iii) les sels d'addition correspondants.

2. Composé oxyimino du thiazole suivant la revendication 1, caractérisé en ce que $R_1$ est H, Cl, Br ou $CH_3$.

3. Composé oxyimino du thiazole suivant la revendication 1, caractérisé en ce que $R_2$ est un groupe $CH_2CH_2NR_5R_6$ dans lequel le groupe $NR_5R_6$ représente un groupe N-hétérocyclique choisi parmi l'ensemble constitué par les groupes pyrrolidino, pipéridino et morpholino.

4. Composé oxyimino du thiazole suivant la revendication 1, caractérisé en ce que $R_2$ est $CH_3$, $C_2H_5$, i-$C_3H_7$, n-$C_4H_9$, $CH_2C_6H_2(R_3)_3$ (où chaque $R_3$, identique ou différent, est H, F, Cl, Br, $CF_3$, $CH_3$, $CH_3O$ ou $C_2H_5O$), $CH_2COONa$, $CH_2COOK$, $CH_2COOCH_3$, $CH_2COOC_2H_5$, $CH_2CH_2N(CH_3)_2$, $CH_2CH_2N(C_2H_5)_2$, 2-pyrrolidinoéthyle, 2-morpholinoéthyle ou 2-pipéridinoéthyle , $R_2$ pouvant représenter H lorsque $R_1$ est différent de H.

5. Composé oxyimino du thiazole suivant la revendication 1, caractérisé en ce que $R_2$ est $CH_3$, $C_2H_5$, i-$C_3H_7$, n-$C_4H_9$, $CH_2C_6H_4R_3$ (où $R_3$ est H, F, Cl, Br, $CF_3$, $CH_3$, $CH_3O$ ou $C_2H_5O$), $CH_2COONa$, $CH_2COOCH_3$, $CH_2COOC_2H_5$, $CH_2CH_2N(CH_3)_2$, $CH_2CH_2N(C_2H_5)_2$ ou 2-pyrrolidinoéthyle.

6. Composé oxyimino du thiazole suivant la revendication 1, caractérisé en ce qu'il est choisi parmi l'ensemble comprenant : (a) l'isomère E et l'isomère Z du 5-(1-méthoxyiminoéthyl)-4-méthylthiazole et les mélanges desdits isomères E et Z ; et, (b) les sels d'addition desdits isomères E et Z et de leurs dits mélanges.

7. Composé oxyimino du thiazole suivant la revendication 6, caractérisé en ce qu'il est constitué par le mélange des isomères E et Z du 5-(1-méthoxyiminoéthyl)-4-méthylthiazole dans un rapport pondéral

E/Z = 91/9.

**8.** Composé oxyimino du thiazole, suivant la revendication 1, caractérisé en ce qu'il est choisi parmi l'ensemble comprenant :
(a) l'isomère E et l'isomère Z de 5-(1-benzyloxyiminoéthyl)-4-méthylthiazole et les mélanges desdits isomères E et Z ; et,
(b) les sels d'addition desdits isomères E et Z et de leurs dits mélanges.

**9.** Composé oxyimino du thiazole suivant la revendication 8, caractérisé en ce qu'il est constitué par le mélange des isomères E et Z du 5-(1-benzyloxyiminoéthyl)-4-méthylthiazole dans un rapport pondéral E/Z = 89/11.

**10.** Composition thérapeutique caractérisée en ce qu'elle renferme, en association avec un excipient physiologiquement acceptable, au moins un composé choisi parmi l'ensemble constitué par
(a) les isomères géométriques E et Z et leurs mélanges de formule

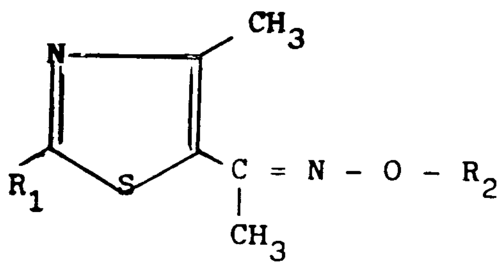

(I bis)

où $R_1$ représente H, F, Cl, Br ou $CH_3$, et $R_2$ représente H, un groupe alkyle en $C_1$-$C_{10}$,
un groupe benzyle de formule $CH_2C_6H_2(R_3)_3$,
où chaque $R_3$, identique ou différent représente H, F, Cl, Br, $CF_3$, un groupe alkyle en $C_1$-$C_4$ ou un groupe alkoxy en $C_1$-$C_4$,
un groupe $CH_2COOR_4$,
où $R_4$ représente H, un groupe alkyle en $C_1$-$C_4$ ou le groupe ammonium $NH_4^+$,
un groupe $CH_2COO1/m$ M,
où M est un métal alcalin ou alcalino-terreux et m sa valence,
ou un groupe 2-aminoéthyle de formule $CH_2CH_2NR_5R_6$,
où $R_5$ et $R_6$, qui peuvent être identiques ou différents, représentent chacun H ou un groupe alkyle en $C_1$-$C_4$, $R_5$ et $R_6$ considérés ensemble avec l'atome d'azote auquel ils sont liés pouvant former un groupe N-hétérocyclique saturé de 5 à 7 sommets et choisi parmi l'ensemble constitué par les groupes pyrrolidino, pipéridino, morpholino, thiomorpholino, et hexaméthylèneimino ; et,
(b) leurs sels d'addition non-toxiques.

**11.** Composition suivant la revendication 10, caractérisée en ce qu'elle renferme au moins un composé oxyimino du thiazole de formule I bis choisi parmi l'ensemble constitué par :
(i) les isomères E et Z et leurs mélanges de formule I bis où $R_1$ est H et $R_2$ est $CH_3$, $C_2H_5$, i-$C_3H_7$, n-$C_4H_9$, $CH_2C_6H_4R_3$ (où $R_3$ est H, F, Cl, Br, $CF_3$, $CH_3$, $CH_3O$ ou $C_2H_5O$), $CH_2COONa$, $CH_2COOCH_3$, $CH_2COOC_2H_5$, $CH_2CH_2N(CH_3)_2$, $CH_2CH_2N(C_2H_5)_2$ ou 2-pyrrolidinoéthyle, et leurs sels d'addition non-toxiques ;
(ii) les isomères E et z et leurs mélanges de formule I bis où $R_1$ est Cl, Br ou $CH_3$ et $R_2$ est H ou $CH_3$, et leurs sels d'addition non-toxiques ; et,
(iii) les isomères E et Z et leurs mélanges de formule I bis où $R_1$ = $R_2$ = H.

**12.** Utilisation thérapeutique d'un composé oxyimino du thiazole, caractérisée en ce que l'on prépare à partir d'une substance choisie parmi les isomères E et Z de 5-(1-alkoxyimino- et 1-hydroxyimino-éthyl)-4-méthylthiazoles de formule I bis et leurs mélanges, d'une part, et leurs sels d'addition non-toxiques, d'autre part, un médicament anticonvulsivant destiné à une utilisation thérapeutique vis-à-vis des convulsions et de l'épilepsie.

**13.** Procédé de préparation d'un composé oxyimino du thiazole de formule I selon la revendication 1, caractérisé en ce que l'on fait réagir, notamment en présence d'acétate de sodium, un composé 5-acétyl-4-méthylthiazole de formule

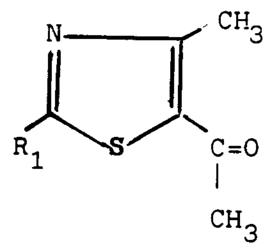

(II)

avec un dérivé d'hydroxylamine de formule :

$H_2NOR_2$ (III)

où $R_1$ et $R_2$ sont définis comme indiqué ci-dessus.

**14.** Procédé de préparation d'un composé oxyimino du thiazole de formule I selon la revendication 1, dans lequel $R_2$ est différent de l'atome d'hydrogène, caractérisé en ce que l'on fait réagir, en présence de triphénylphosphine et d'azodicarboxylate de diéthyle, un composé hydroxyimino du thiazole de formule

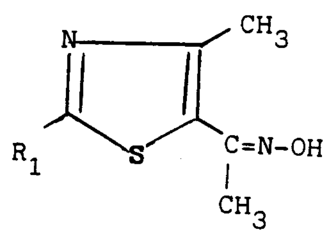

(I')

où $R_1$ est défini comme indiqué ci-dessus, avec un alcool

$R_2OH$ (IV)

où $R_2$ est défini comme indiqué dans la revendication 1 mais différent de H.

**Revendications pour les Etats contractants suivants : ES, GR**

**1.** Procédé de préparation d'un composé oxyimino du thiazole choisi parmi l'ensemble comprenant
(i) les 5-(1-alkoxyimino- et 1-hydroxyimino-éthyl)-4-méthylthiazoles de formule

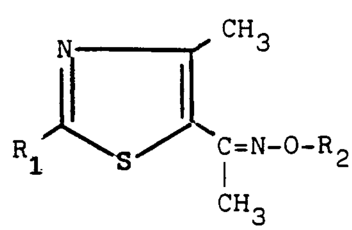

(I)

dans laquelle $R_1$ représente H, F, Cl, Br, ou $CH_3$ ; et, $R_2$ représente un groupe alkyle en $C_1$-$C_{10}$, un groupe benzyle de formule $CH_2C_6H_2(R_3)_3$,
où chaque $R_3$, identique ou différent, représente H, F, Cl, Br, $CF_3$ un groupe alkyle en $C_1$-$C_4$ ou

un groupe alkoxy en $C_1$-$C_4$,
un groupe $CH_2COOR_4$,
où $R_4$ représente H, un groupe alkyle en $C_1$-$C_4$ ou le groupe ammonium $NH_4^+$,
un groupe $CH_2COO1/m$ M,
où M est un métal alcalin ou alcalino-terreux et m sa valence,
ou un groupe 2-aminoéthyle de formule $CH_2CH_2NR_5R_6$,
où $R_5$ et $R_6$, qui peuvent être identiques ou différents, représentent chacun H ou un groupe alkyle en $C_1$-$C_4$, $R_5$ et $R_6$ considérés ensemble avec l'atome d'azote auquel ils sont liés pouvant former un groupe N-hétérocyclique saturé de 5 à 7 sommets et choisi parmi l'ensemble constitué par les groupes pyrrolidino, pipéridino, morpholino, thiomorpholino, et hexaméthylèneimino $R_2$ pouvant également représenter l'atome d'hydrogène quand $R_1$ est différent de H ;
(ii) leurs isomères géométriques E et Z ; et,
(iii) les sels d'addition correspondants,

ledit procédé étant caractérisé en ce que l'on fait réagir, notamment en présence d'acétate de sodium, un composé 5-acétyl-4-méthylthiazole de formule

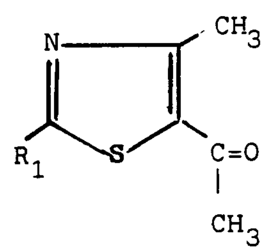

(II)

avec un dérivé d'hydroxylamine de formule :

$H_2NOR_2$ (III)

où $R_1$ et $R_2$ sont définis comme indiqué ci-dessus.

**2.** Procédé suivant la revendication 1 pour la préparation d'un composé oxyimino du thiazole de formule I où $R_2$ est différent de H, ledit procédé étant caractérisé en ce que l'on remplace le composé de formule II par un composé hydroxyimino du thiazole de formule

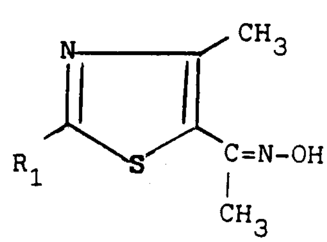

(I')

où $R_1$ est défini comme indiqué ci-dessus, d'une part, et le composé de formule III par un alcool

$R_2OH$ (IV)

où $R_2$ est défini comme indiqué dans la revendication 1 mais différent de H.

**3.** Procédé suivant la revendication 2, dans lequel la réaction de I' avec IV est réalisée en présence de triphénylphosphine et d'azodicarboxylate de diéthyle.

**4.** Procédé suivant la revendication 1 ou 2, dans lequel $R_1$ est H, Cl, Br ou $CH_3$.

**5.** Procédé suivant la revendication 1 ou 2, dans lequel le groupe $NR_5R_6$ représente un groupe N-hétérocyclique choisi parmi l'ensemble constitué par les groupes pyrrolidino, pipéridino et morpholino.

**6.** Procédé suivant la revendication 1, dans lequel $R_2$ est $CH_3$, $C_2H_5$, i-$C_3H_7$, n-$C_4H_9$, $CH_2C_6H_2(R_3)_3$ (où chaque $R_3$, identique ou différent, est H, F, Cl, Br, $CF_3$, $CH_3$, $CH_3O$ ou $C_2H_5O$), $CH_2COONa$, $CH_2COOK$, $CH_2COOCH_3$, $CH_2COOC_2H_5$, $CH_2CH_2N(CH_3)_2$, $CH_2CH_2N(C_2H_5)_2$, 2-pyrrolidinoéthyle, 2-morpholinoéthyle ou 2-pipéridinoéthyle , $R_2$ pouvant représenter H lorsque $R_1$ est différent de H.

**7.** Procédé suivant la revendication 1 ou 2, dans lequel $R_2$ est $CH_3$, $C_2H_5$, i-$C_3H_7$, n-$C_4H_9$, $CH_2C_6H_4R_3$ - (où $R_3$ est H, F, Cl, Br, $CF_3$, $CH_3$, $CH_3O$ ou $C_2H_5O$), $CH_2COONa$, $CH_2COOCH_3$, $CH_2COOC_2H_5$, $CH_2CH_2N(CH_3)_2$, $CH_2CH_2N(C_2H_5)_2$ ou 2-pyrrolidinoéthyle.

**Claims**

**Claims for the following Contracting States : AT, BE, CH, DE, FR, GB, IT, LI, LU, NL, SE**

**1.** An oxyiminothiazole compound characterized in that it is selected from the group consisting of

(i) the 5-(1-alkoxyimino- and 1-hydroxyimino-ethyl)-4- methylthiazoles of the formula wherein,

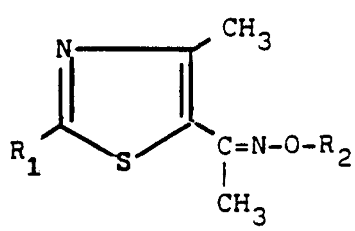

(I)

$R_1$ represents H, F, Cl, Br or $CH_3$ ; and

$R_2$ represents

a $C_1$-$C_{10}$ alkyl group,

a benzyl group of the formula $CH_2C_6H_2(R_3)_3$

in which each $R_3$, identical or different represents H, F, Cl, Br, $CF_3$, a $C_1$-$C_4$ alkyl group or a $C_1$-$C_4$ alkoxy group,

a $CH_2COOR_4$ group,

in which $R_4$ represents H, a $C_1$-$C_4$ alkyl group or $NH_4^+$,

a $CH_2COO^1/mM$ group,

in which M is an alkali metal or alkaline earth metal and m is its valency,

or a 2-aminoethyl group of the formula $CH_2CH_2NR_5$

$R_6$, in which $R_5$ and $R_6$, which can be identical or different, each represent H or a $C_1$-$C_4$ alkyl group, $R_5$ and $R_6$, taken together with the nitrogen atom to which they are bonded being able to form a 5- to 7-membered N-heterocyclic group selected from the group consisting of pyrrolidino, piperidino, morpholino and hexamethylene-imino,

$R_2$ being able to represent the hydrogen atom when

$R_1$ is other than H ;

(ii) the E and Z geometrical isomers thereof ; and

(iii) the corresponding addition salts.

**2.** An oxyiminothiazole compound according to claim 1 wherein $R_1$ is H, Cl, Br or $CH_3$.

**3.** An oxyiminothiazole compound according to claim 1 wherein $R_2$ is a group $CH_2CH_2NR_5R_6$ in which the group $NR_5R_6$ represents an N-heterocyclic group selected from the group consisting of the pyrrolidino, piperidino and morpholino groups.

**4.** An oxyiminothiazole compound according to claim 1 wherein $R_2$ is $CH_3$, $C_2H_5$, i-$C_3H_7$, n-$C_4H_9$, $CH_2C_6H_2(R_3)_3$ (in which each $R_3$, identical or different, is H, F, Cl, Br, $CF_3$, $CH_3$, $CH_3O$ or $C_2H_5O$), $CH_3COONa$, $CH_3COOK$, $CH_3COOCH_3$,$CH_3COOC_2H_5$, $CH_2CH_2N(CH_3)_2$, $CH_2CH_2N(C_2H_5)_2$, 2-pyrrolidinoethyl, 2-morpholinoethyl or 2-piperidinoethyl, $R_2$ being able to represent H when $R_1$ is different from H.

**5.** An oxyiminothiazole compound according to claim 1 wherein $R_2$ is $CH_3$, $C_2H_5$, i-$C_3H_7$, n-$C_4H_9$, $CH_2C_6H_4R_3$ (in which $R_3$ is H, F, Cl, Br, $CF_3$, $CH_3$, $CH_3O$ or $C_2H_5O$), $CH_3COONa$, $CH_3COOCH_3$, $CH_3COOC_2H_5$, $CH_2CH_2N(CH_3)_2$, $CH_2CH_2N(C_2H_5)_2$ or 2-pyrrolidinoethyl.

**6.** An oxyiminothiazole compound according to claim 1 which is selected from the group consisting of :
(a) the E isomer and the Z isomer of 5-(1-methoxyiminoethyl)-4-methylthiazoleand mixtures of said E and Z isomers ; and
(b) the addition salts of the said E and Z isomers and of said mixtures thereof.

**7.** An oxyiminothiazole compound according to claim 6, which consists of a mixture of the E and Z isomers of 5-(1-methoxyiminoethyl)-4-methylthiazole in which the weight ratio E/Z is 91/9.

**8.** An oxyiminothiazole compound according to claim 1 which is selected from the group consisting of :
(a) the E isomer and the Z isomer of 5-(1-benzyloxyiminoethyl)-4-methylthiazole and mixtures of said E and Z isomers ; and
(b) the addition salts of the said E and Z isomers and of said mixtures thereof.

**9.** An oxyiminothiazole compound according to claim 8 which consists of a mixture of the E and Z isomers of 5-(1-benzyloxyiminoethyl)-4-methylthiazole in which the weight ratio E/Z is 89/11.

**10.** A therapeutic composition which contains, in association with a physiologically acceptable excipient, at least one compound selected from the group consisting of :
(a) the E and Z geometrical isomers and mixtures thereof of the formula

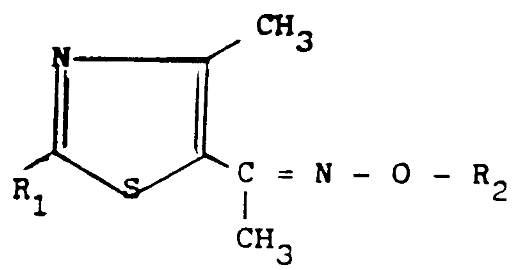

(I bis)

wherein,
$R_1$ represents H, F, Cl, Br or $CH_3$ ; and
$R_2$ represents
a $C_1$-$C_{10}$ alkyl group,
a benzyl group of the formula $CH_2C_6H_2(R_3)_3$ in which each $R_3$, identical or different represents H, F, Cl, Br, $CF_3$, a $C_1$-$C_4$ alkyl group or a $C_1$-$C_4$ alkoxy group,
a $CH_2COOR_4$ group,
in which $R_4$ represents H, a $C_1$-$C_4$ alkyl group or $NH_4^+$,
a $CH_2COO^1/mM$ group,
in which M is an alkali metal or alkaline earth metal and m is its valency,
or a 2-aminoethyl group of the formula $CH_2CH_2NR_5R_6$, in which $R_5$ and $R_6$, which can be identical or different, each represent H or a $C_1$-$C_4$ alkyl group, $R_5$ and $R_6$, taken together with the nitrogen atom to which they are bonded being able to form a 5- to 7-membered N-heterocyclic group selected from the group consisting of pyrrolidino, piperidino, morpholino and hexamethylene-imino,
$R_2$ being able to represent the hydrogen atom when
$R_1$ is other than H ; and,
(b) non-toxic addition salts thereof.

**11.** A composition according to claim 10 which contains at least one oxyiminothiazole compound of formula I bis selected from the group consisting of :
(i) the E and Z isomers and mixtures thereof of formula I bis in which $R_1$ is H and $R_2$ is $CH_3$, $C_2H_5$, i-$C_3H_7$, n-$C_4H_9$, $CH_2C_6H_4R_3$ (in which $R_3$ is H, F, Cl, Br, $CF_3$, $CH_3$, $CH_3O$ or $C_2H_5O$), $CH_3COONa$,

$CH_3COOCH_3$, $CH_3COOC_2H_5$, $CH_2CH_2N(CH_3)_2$, $CH_2CH_2N(C_2H_5)_2$ or 2-pyrrolidinoethyl, and non-toxic addition salts thereof ;
(ii) the E and Z isomers and mixtures thereof of formula I bis in which $R_1$ is Cl, Br or $CH_3$ and $R_2$ is H or $CH_3$, and non-toxic addition salts thereof ; and
(iii) the E and Z isomers and mixtures thereof of formula I bis in which $R_1 = R_2 = H$.

**12.** A therapeutic use of an oxyiminothiazole compound, wherein an anticonvulsant drug for use in therapy in the treatment of convulsions and epilepsy is prepared from a substance selected on the one hand from the E and Z isomers of the 5-(1-alkoxyimino- and 1-hydroxyimino-ethyl)-4-methylthiazoles of formula I bis and mixtures thereof, and on the other hand from the non-toxic addition salts thereof.

**13.** A method for the preparation of an oxyiminothiazole compound of formula I according to claim 1, wherein a 5-acetyl-4-methylthiazole compound of the formula

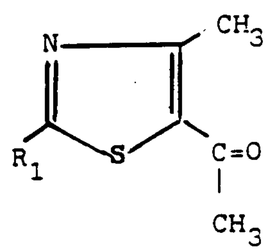

(II)

is reacted, especially in the presence of sodium acetate, with a hydroxylamine compound of the formula

$H_2NOR_2$ (III)

in which $R_1$ and $R_2$ are defined as indicated above.

**14.** A method for the preparation of an oxyiminothiazole compound of formula I according to claim 1 in which $R_2$ is other than the hydrogen atom, wherein a hydroxyiminothiazole compound of the formula

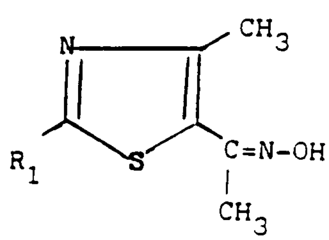

(I')

in which $R_1$ is defined as indicated above, is reacted, especially in the presence of triphenylphosphine and diethyl azodicarboxylate, with an alcohol of the formula

$R_2OH$ (IV)

in which $R_2$ is defined as indicated in claim 1, but different from H.

**Claims for the following Contracting States : ES, GR**

1. A method for the preparation of an oxyiminothiazole compound selected from the group consisting of
   (i) the 5-(1-alkoxyimino- and 1-hydroxyimino-ethyl)-4- methylthiazoles of the formula wherein,

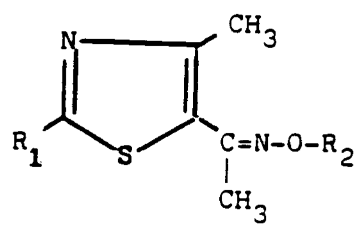

(I)

$R_1$ represents H, F, Cl, Br or $CH_3$ ; and
$R_2$ represents
a $C_1$-$C_{10}$ alkyl group,
a benzyl group of the formula $CH_2C_6H_2(R_3)_3$
in which each $R_3$, identical or different represents H, F, Cl, Br, $CF_3$, a $C_1$-$C_4$ alkyl group or a $C_1$-$C_4$ alkoxy group,
a $CH_2COOR_4$ group,
in which $R_4$ represents H, a $C_1$-$C_4$ alkyl group or $NH_4^+$,
a $CH_2COO^1/mM$ group,
in which M is an alkali metal or alkaline earth metal and m is its valency,
or a 2-aminoethyl group of the formula $CH_2CH_2NR_5R_6$,
in which $R_5$ and $R_6$, which can be identical or different, each represent H or a $C_1$-$C_4$ alkyl group, $R_5$ and $R_6$, taken together with the nitrogen atom to which they are bonded being able to form a 5- to 7-membered N-heterocyclic group selected from the group consisting of pyrrolidino, piperidino, morpholino and hexamethylene-imino,
$R_2$ being able to represent the hydrogen atom when
$R_1$ is other than H ;

(ii) the E and Z geometrical isomers thereof ; and
(iii) the corresponding addition salts ; said method comprising reacting a 5-acetyl-4-methylthiazole compound of the formula

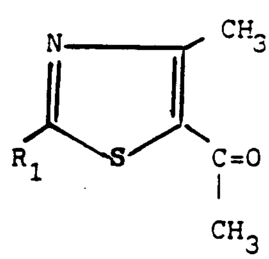

(II)

especially in the presence of sodium acetate, with a hydroxylamine compound of the formula

$H_2NOR_2$ (III)

in which $R_1$ and $R_2$ are defined as indicated above.

2. A method for the preparation of an oxyiminothiazole compound of formula I according to claim 1 in which $R_2$ is other than the hydrogen atom, wherein a hydroxyiminothiazole compound of the formula

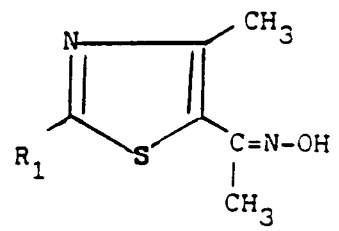

(I')

in lieu of the compound of the formula II, is reacted with an alcohol of the formula

$R_2OH$ (IV)

in which $R_2$ is defined as indicated in claim 1, but different from H.

**3.** A method according to claim 2, wherein the reaction of I' with IV is carried out in the presence of triphenylphosphine and diethyl azodicarboxylate.

**4.** A method according to claim 1 or 2, wherein $R_1$ is H, Cl, Br or $CH_3$.

**5.** A method according to claim 1 or 2, wherein the group $NR_5R_6$ represents an N-heterocyclic group selected from the group consisting of the pyrrolidino, piperidino and morpholino groups.

**6.** A method according to claim 1, wherein $R_2$ is $CH_3$, $C_2H_5$, i-$C_3H_7$, n-$C_4H_9$, $CH_2C_6H_2(R_3)_3$ (in which each $R_3$, identical or different, is H, F, Cl, Br, $CF_3$, $CH_3$, $CH_3O$ or $C_2H_5O$), $CH_3COONa$, $CH_3COOK$, $CH_3COOCH_3$, $CH_3COOC_2H_5$, $CH_2CH_2N(CH_3)_2$, $CH_2CH_2N(C_2H_5)_2$, 2-pyrrolidinoethyl, 2-morpholinoethyl or 2-piperidinoethyl, $R_2$ being able to represent H when $R_1$ is different from H.

**7.** A method according to claim 1 or 2, wherein $R_2$ is $CH_3$, $C_2H_5$, i-$C_3H_7$, n-$C_4H_9$, $CH_2C_6H_4R_3$ (in which $R_3$ is H, F, Cl, Br, $CF_3$, $CH_3$, $CH_3O$ or $C_2H_5O$), $CH_3COONa$, $CH_3COOCH_3$, $CH_3COOC_2H_5$, $CH_2CH_2N$-$(CH_3)_2$, $CH_2CH_2N$ $(C_2H_5)_2$ or 2-pyrrolidinoethyl.

**Patentansprüche**

**Patentansprüche für folgende Vertragsstaaten : AT, BE, CH, DE, FR, GB, IT, LI, LU, NL, SE**

**1.** Oxyimino-Verbindung des Thiazols, dadurch gekennzeichnet, daß sie ausgewählt ist aus der Gruppe, die enthält

(i) die 5-(1-Alkoxyimino- und 1-Hydroxyimino-ethyl)-4-methylthiazole der Formel

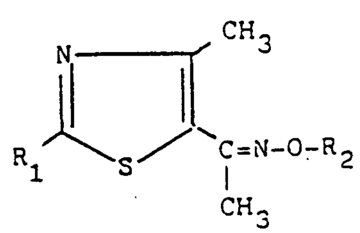

(I)

in der $R_1$ H, F, Cl, Br oder $CH_3$ darstellt; und

$R_2$ eine $C_1$-$C_{10}$-Alkylgruppe,

eine Benzylgruppe der Formel $CH_2C_6H_2(R_3)_3$,

worin jedes $R_3$, gleich oder verschieden, H, F, Cl, Br, $CF_3$, eine $C_1$-$C_4$-Alkylgruppe oder eine $C_1$-$C_4$-Alkoxygruppe darstellt,

eine $CH_2COOR_4$-Gruppe,

worin $R_4$ H, eine $C_1$-$C_4$-Alkylgruppe oder die Ammoniumgruppe $NH_4^+$ darstellt,

eine Gruppe $CH_2COO1/m$ M,

worin M ein Alkali- oder Erdalkalimetall und m seine Valenz ist,
oder eine 2-Aminoethylgruppe der Formel $CH_2CH_2NR_5R_6$,
worin $R_5$ und $R_6$, die gleich oder verschieden sein können, jeweils H oder eine $C_1$-$C_4$-Alkylgruppe darstellen, wobei $R_5$ und $R_6$ zusammen mit dem Stickstoffatom, an das sie gebunden sind, betrachtet einen gesättigten 5- bis 7-gliedrigen N-heterocyclischen Rest bilden können und aus der aus Pyrrolidino-, Piperidino-, Morpholino-, Thiomorpholino- und Hexamethyleniminoresten bestehenden Gruppe ausgewählt sind,
darstellt, wobei $R_2$ auch das Wasserstoffatom darstellen kann, wenn $R_1$ von H verschieden ist;
(ii) deren geometrische E- und Z-Isomere; und
(iii) die entsprechenden Additionssalze.

**2.** Oxyimino-Verbindung des Thiazols nach Anspruch 1, dadurch gekennzeichnet, daß $R_1$ H, Cl, Br oder $CH_3$ ist.

**3.** Oxyimino-Verbindung des Thiazols nach Anspruch 1, dadurch gekennzeichnet, daß $R_2$ eine $CH_2CH_2NR_5R_6$-Gruppe ist, in welcher der Rest $NR_5R_6$ einen N-heterocyclischen Rest darstellt, welcher aus der aus den Pyrrolidino-, Piperidino- und Morpholinoresten bestehenden Gruppe ausgewählt ist.

**4.** Oxyimino-Verbindung des Thiazols nach Anspruch 1, dadurch gekennzeichnet-, daß $R_2$ $CH_3$, $C_2H_5$, i-$C_3H_7$, n-$C_4H_9$, $CH_2C_6H_2(R_3)_3$ (worin jedes $R_3$, gleich oder verschieden, H, F, Cl, Br, $CF_3$, $CH_3$, $CH_3O$ oder $C_2H_5O$ ist), $CH_2COONa$, $CH_2COOK$, $CH_2COOCH_3$, $CH_2COOC_2H_5$, $CH_2CH_2N(CH_3)_2$, $CH_2CH_2N$-$(C_2H_5)_2$, 2-Pyrrolidinoethyl, 2-Morpholinoethyl oder 2-Piperidinoethyl ist, wobei $R_2$ H darstellen kann, wenn $R_1$ von H verschieden ist.

**5.** Oxyimino-Verbindung des Thiazols nach Anspruch 1, dadurch gekennzeichnet, daß $R_2$ $CH_3$, $C_2H_5$, i-$C_3H_7$, n-$C_4H_9$, $CH_2C_6H_4R_3$ (worin $R_3$ H, F, Cl, Br, $CF_3$, $CH_3$, $CH_3O$ oder $C_2H_5O$ ist), $CH_2COONa$, $CH_2COOCH_3$, $CH_2COOC_2H_5$,$CH_2CH_2N(CH_3)_2$, $CH_2CH_2N(C_2H_5)_2$ oder 2-Pyrrolidinoethyl ist.

**6.** Oxyimino-Verbindung des Thiazols nach Anspruch 1, dadurch gekennzeichnet, daß sie ausgewählt ist aus der Gruppe, die (a) das E-Isomer und das Z-Isomer des 5-(1-Methoxyiminoethyl)-4-methylthiazols und die Mischungen besagter E- und Z-Isomerer; und (b) die Additionssalze besagter E- und Z-Isomerer und ihrer besagten Mischungen enthält.

**7.** Oxyimino-Verbindung des Thiazols nach Anspruch 6, dadurch gekennzeichnet, daß sie aus der Mischung der E- und Z-Isomeren des 5-(1-Methoxyiminoethyl)-4-methylthiazols in einem Gewichtsverhältnis E/Z = 91/9 besteht.

**8.** Oxyimino-Verbindung des Thiazols nach Anspruch 1, dadurch gekennzeichnet, daß sie ausgewählt ist aus der Gruppe, die (a) das E-Isomer und das Z-Isomer des 5-(1-Benzyloxyiminoethyl)-4-methylthiazols und die Mischungen besagter E- und Z-Isomerer; und (b) die Additionssalze besagter E- und Z-Isomerer und ihrer besagten Mischungen enthält.

**9.** Oxyimino-Verbindung des Thiazols nach Anspruch 8, dadurch gekennzeichnet, daß sie aus der Mischung der E- und Z-Isomeren des 5-(1-Benzyloxyiminoethyl)-4-methylthiazols in einem Gewichtsverhältnis E/Z = 89/11 besteht.

**10.** Therapeutische Zusammensetzung, dadurch gekennzeichnet, daß sie in Verbindung mit einem physiologisch annehmbaren Streckmittel mindestens eine Verbindung einschließt, die ausgewählt ist aus der Gruppe, die besteht aus

(a) den geometrischen E- und Z-Isomeren und deren Mischungen der Formel

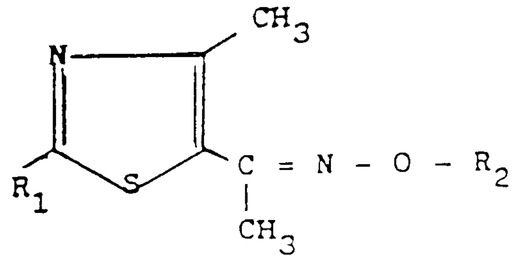

(I bis)

worin $R_1$ H, F, Cl, Br oder $CH_3$ darstellt, und $R_2$ H, eine $C_1$-$C_{10}$-Alkylgruppe,
eine Benzylgruppe der Formel $CH_2C_6H_2(R_3)_3$,
worin jedes $R_3$, gleich oder verschieden, H, F, Cl, Br, $CF_3$, eine $C_1$-$C_4$-Alkylgruppe oder eine $C_1$-$C_4$-Alkoxygruppe darstellt,
eine $CH_2COOR_4$-Gruppe,
worin $R_4$ H, eine $C_1$-$C_4$-Alkylgruppe oder die Ammoniumgruppe $NH_4^+$ darstellt,
eine Gruppe $CH_2COO1/m$ M,
worin M ein Alkali- oder Erdalkalimetall und m seine Valenz ist,
oder eine 2-Aminoethylgruppe der Formel $CH_2CH_2NR_5R_6$,
worin $R_5$ und $R_6$, die gleich oder verschieden sein können, jeweils H oder eine $C_1$-$C_4$-Alkylgruppe darstellen, wobei $R_5$ und $R_6$ zusammen mit dem Stickstoffatom, an das sie gebunden sind, betrachtet einen gesättigten 5- bis 7-gliedrigen N-heterocyclischen Rest bilden können und aus der aus Pyrrolidino-, Piperidino-, Morpholino-, Thiomorpholino- und Hexamethyleniminoresten bestehenden Gruppe ausgewählt sind,
darstellt; und
(b) ihren nicht-toxischen Additionssalzen.

**11.** Zusammensetzung nach Anspruch 10, dadurch gekennzeichnet, daß sie mindestens eine Oxyimino-Verbindung des Thiazols der Formel I bis einschließt, die ausgewählt ist aus der Gruppe, die besteht aus:
(i) den E- und Z-Isomeren und deren Mischungen der Formel I bis, worin $R_1$ H ist und $R_2$ $CH_3$, $C_2H_5$, i-$C_3H_7$, n-$C_4H_9$, $CH_2C_6H_4R_3$ (worin $R_3$ H, F, Cl, Br, $CF_3$, $CH_3$, $CH_3O$ oder $C_2H_5O$ ist), $CH_2COONa$, $CH_2COOCH_3$, $CH_2COOC_2H_5$,$CH_2CH_2N(CH_3)_2$, $CH_2CH_2N(C_2H_5)_2$ oder 2-Pyrrolidinoethyl ist, und deren nicht-toxischen Additionssalzen;
(ii) den E- und Z-Isomeren und deren Mischungen der Formel I bis, worin $R_1$ Cl, Br oder $CH_3$ ist und $R_2$ H oder $CH_3$ ist, und deren nicht-toxischen Additionssalzen; und
(iii) den E- und Z-Isomeren und deren Mischungen der Formel I bis, worin $R_1$ = $R_2$ = H.

**12.** Therapeutische Verwendung einer Oxyimino-Verbindung des Thiazols, dadurch gekennzeichnet, daß man ausgehend von einer Substanz, die ausgewählt ist aus den E- und Z-Isomeren von 5-(1-Alkoxyimino- und 1-Hydroxyimino-ethyl)-4-methylthiazolen der Formel I bis und deren Mischungen einerseits und deren nicht-toxischen Additionssalzen andererseits, ein krampfhemmendes Medikament, das für eine therapeutische Anwendung gegen Krämpfe und Epilepsie bestimmt ist, herstellt.

**13.** Verfahren zur Herstellung einer Oxyimino-Verbindung des Thiazols der Formel I nach Anspruch 1, dadurch gekennzeichnet, daß man eine 5-Acetyl-4-methylthiazol-Verbindung der Formel

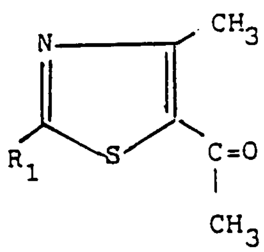

(II)

mit einem Hydroxylaminderivat der Formel:

$H_2NOR_2$ (III)

worin $R_1$ und $R_2$ wie oben angezeigt definiert sind, insbesondere in Gegenwart von Natriumacetat reagieren läßt.

**14.** Verfahren zur Herstellung einer Oxyimino-Verbindung des Thiazols der Formel I nach Anspruch 1, in der $R_2$ vom Wasserstoffatom verschieden ist, dadurch gekennzeichnet, daß man eine Hydroxyimino-Verbindung des Thiazols der Formel

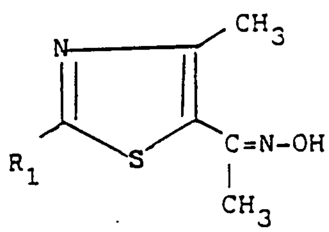

(I')

worin $R_1$ wie oben angezeigt definiert ist, mit einem Alkohol

$R_2OH$ (IV)

worin $R_2$ wie im Anspruch 1 angezeigt definiert, aber von H verschieden ist, in Gegenwart von Triphenylphosphin und Diethylazodicarboxylat reagieren läßt.

**Patentansprüche für folgende Vertragsstaaten : ES, GR**

**1.** Verfahren zur Herstellung einer Oxyimino-Verbindung des Thiazols, die aus der Gruppe ausgewält ist, welche umfasst

(i) die 5-(1-Alkoxyimino- und 1-Hydroxyimino-ethyl)-4-methylthiazole der Formel

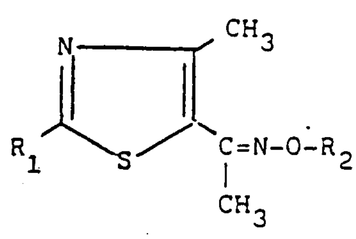

(I)

in der $R_1$ H, F, Cl, Br oder $CH_3$ darstellt; und

$R_2$ eine $C_1$-$C_{10}$-Alkylgruppe,

eine Benzylgruppe der Formel $CH_2C_6H_2(R_3)_3$,

worin jedes $R_3$, gleich oder verschieden, H, F, Cl, Br, $CF_3$, eine $C_1$-$C_4$-Alkylgruppe oder eine $C_1$-$C_4$-Alkoxygruppe darstellt,

eine $CH_2COOR_4$-Gruppe,

worin $R_4$ H, eine $C_1$-$C_4$-Alkylgruppe oder die Ammoniumgruppe $NH_4^+$ darstellt,

eine Gruppe $CH_2COO1/m$ M,

worin M ein Alkali- oder Erdalkalimetall und m seine Valenz ist,

oder eine 2-Aminoethylgruppe der Formel $CH_2CH_2NR_5R_6$,

worin $R_5$ und $R_6$, die gleich oder verschieden sein können, jeweils H oder eine $C_1$-$C_4$-Alkylgruppe darstellen, wobei $R_5$ und $R_6$ zusammen mit dem Stickstoffatom, an das sie gebunden sind, betrachtet einen gesättigten 5- bis 7-gliedrigen N-heterocyclischen Rest bilden können und aus der aus Pyrrolidino-, Piperidino-, Morpholino-, Thiomorpholino- und Hexamethyleniminoresten bestehenden Gruppe ausgewählt sind,

darstellt, wobei $R_2$ auch das Wasserstoffatom darstellen kann, wenn $R_1$ von H verschieden ist;

(ii) deren geometrische E- und Z-Isomere; und

(iii) die entsprechenden Additionssalze,

wobei das besagte Verfahren dadurch gekennzeichnet ist, daß man eine 5-Acetyl-4-methylthiazol-Verbindung der Formel

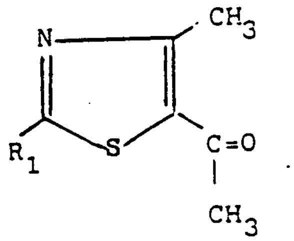

(II)

mit einem Hydroxylaminderivat der Formel:

$H_2NOR_2$ (III)

worin $R_1$ und $R_2$ wie oben angezeigt definiert sind, insbesondere in Gegenwart von Natriumacetat reagieren läßt.

2. Verfahren nach Anspruch 1 für die Herstellung einer Oxyimino-Verbindung des Thiazols der Formel I, worin $R_2$ von H verschieden ist, wobei das besagte Verfahren dadurch gekennzeichnet ist, daß man einerseits die Verbindung der Formel II durch einen Hydroxyimino-Verbindung des Thiazols der Formel

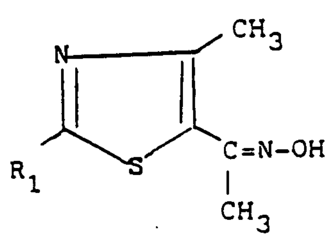

(I')

worin $R_1$ wie oben angezeigt definiert ist, und die Verbindung der Formel III durch einen Alkohol

$R_2OH$ (IV)

worin $R_2$ wie im Anspruch 1 angezeigt, aber von H verschieden definiert ist, ersetzt.

3. Verfahren nach Anspruch 2, in dem die Reaktion von I' mit IV in Gegenwart von Triphenylphosphin und Diethylazodicarboxylat durchgeführt wird.

4. Verfahren nach Anspruch 1 oder 2, in dem $R_1$ H, Cl, Br oder $CH_3$ ist.

5. Verfahren nach Anspruch 1 oder 2, in dem der Rest $NR_5R_6$ einen N-heterocyclischen Rest darstellt, der aus der aus den Pyrrolidino-, Piperidino- und Morpholinoresten bestehenden Gruppe ausgewählt ist.

6. Verfahren nach Anspruch 1, in dem $R_2$ $CH_3$, $C_2H_5$, i-$C_3H_7$, n-$C_4H_9$, $CH_2C_6H_2(R_3)_3$ (worin jedes $R_3$, gleich oder verschieden, H, F, Cl, Br, $CF_3$, $CH_3$, $CH_3O$ oder $C_2H_5O$ ist), $CH_2COONa$, $CH_2COOK$, $CH_2COOCH_3$, $CH_2COOC_2H_5$, $CH_2CH_2N(CH_3)_2$, $CH_2CH_2N(C_2H_5)_2$, 2-Pyrrolidinoethyl, 2-Morpholinoethyl oder 2-Piperidinoethyl ist, wobei $R_2$ H darstellen kann, wenn $R_1$ von H verschieden ist.

7. Verfahren nach Anspruch 1 oder 2, in dem $R_2$ $CH_3$, $C_2H_5$, i-$C_3H_7$, n-$C_4H_9$, $CH_2C_6H_4R_3$ (worin $R_3$ H, F, Cl, Br, $CF_3$, $CH_3$, $CH_3O$ oder $C_2H_5O$ ist), $CH_2COONa$, $CH_2COOCH_3$, $CH_2COOC_2H_5$, $CH_2CH_2N(CH_3)_2$, $CH_2CH_2N(C_2H_5)_2$ oder 2-Pyrrolidinoethyl ist.